# EUROPEAN PATENT APPLICATION

(11) **EP 2 005 961 A1**
(43) Date of publication of application: **24.12.2008**
(21) Application number: 07110868.2
(22) Date of filing: 22.06.2007
(51) Int. Cl.: A61K 31/506, A61P 35/02

(54) **Method of optimizing the treatment of chronic myeloid leukemia with Abl tyrosine kinase inhibitors**

(71) Applicant: MEDVET SCIENCE PTY. LTD., Stepney, South Australia 5069 (AU)
(72) Inventor: The designation of the inventor has not yet been filed
(74) Representative: Roth, Peter Richard

(57) **Abstract**

The present invention relates to a method of treating chronic myeloid leukemia (CML) in a human patient population. More specifically, the method comprises the steps of (a) determining the OCT-1 Activity in pre-therapy blood of a warm-blooded animal suffering from CML, and (b) administering a daily dose between about 500 and 1200 mg of Imatinib mesylate to the warm-blooded animal suffering from CML showing an OCT-1 Activity below about 6.0 to 10.0 ng/200,000 cells, especially about 8.0 to 8.5 ng/200,000 cells.

## Description

The present invention relates to a method of treating chronic myeloid leukemia (CML) in a human patient population.

The success of treatment with Imatinib mesylate in the majority of chronic phase CML patients is well established. Improving treatment outcomes for those patients who perform less well however requires a detailed understanding of the critical determinants of treatment response. It was previously demonstrated that intrinsic sensitivity to Imatinib-induced kinase inhibition is a good predictor of response (White D, Saunders V, Lyons AB, et al. Blood. 2005;106:2520-2526; Schultheis B, Szydlo R, Mahon FX, Apperley JF, Melo JV. Blood. 2005;105:4893-4894) and that this is closely related to the intracellular uptake and retention (IUR) of imatinib (White DL, Saunders VA, Dang P, et al. Blood. 2006;108:697-704). Further it has been demonstrated that the active transport of imatinib is dependent on the organic cation transporter OCT-1 (Thomas J, Wang L, Clark RE, Pirmohamed M. Blood. 2004;104:3739-3745; White DL, Saunders VA, Dang P, et al, OCT-1 mediated influx is a key determinant of the intracellular uptake of imatinib but not nilotinib. Blood. 2006; 108: 697-704).

The OCT-1 protein is a member of the largest superfamily of transporters, the solute carrier family (Koepsell H, Endou H. Pflugers Arch. 2004;447:666-676) which transport in an electrogenic fashion a variety of organic cations, including drugs, toxins and other xenobiotics. The transporter is predicted to have 12 transmembrane domains, and binding pockets with partially overlapping interaction domains for different substrates and inhibitors (Koepsell H, Schmitt BM, Gorboulev V. Rev Physiol Biochem Pharmacol. 2003;150:36-90). Post transcriptional regulation of OCT-1 by phosphorylation status (Ciarimboli G, Schlatter E. Pflugers Arch. 2005;449:423-441) and such compounds as PKA, Src-like p56 and CaM have also been demonstrated.

Surprisingly, it was now found that a significant correlation exists between the IC50^{imatinib} and OCT-1 Activity, and that patients with a low IC50^{imatinib} have significantly greater OCT-1 Activity than patients with high IC50^{imatinib}. By comparing the OCT-1 Activity with molecular response in all patients enrolled to the TIDEL trial (chronic phase newly diagnosed CML patients who received 600 mg Imatinib mesylate up-front), it was demonstrated that patients with high OCT-1 Activity achieve significantly greater molecular responses over 24 months of Imatinib treatment than patients with low OCT-1 Activity. Further it was found that the molecular response of those patients with low OCT-1 Activity is highly dose dependent, with patients receiving 600 mg of Imatinib mesylate achieving significantly better molecular response by 24 months. Significantly, a group of patients with low OCT-1 Activity was identified who are at higher risk for suboptimal or failed Imatinib response if they do not receive the trial dose of 600 mg of Imatinib mesylate per day. The data disclosed herein demonstrates that dose is an important factor for overcoming suboptimal response in low OCT-1 Activity patients.

Importantly, using this functional assay to determine OCT-1 Activity at the time of diagnosis may identify CML patients likely to respond well to standard dose Imatinib mesylate, and those who would be most likely to benefit from a higher dose of Imatinib mesylate.

Hence, the present invention pertains to a method of treating CML in a warm-blooded animal comprising the steps of
(a) determining the OCT-1 Activity in pre-therapy blood of a warm-blooded animal suffering from CML, and
(b) administering a daily dose between about 500 and 1200 mg of Imatinib mesylate to the warm-blooded animal suffering from CML showing an OCT-1 Activity below about 6.0 to 10.0 ng/200,000 cells, especially about 8.0 to 8.5 ng/200,000 cells.

Preferably, the warm blooded animal is a human.

In a preferred embodiment, the daily dose to be administered is between about 600 and 1000 mg of Imatinib mesylate, e.g. 600 mg/day or 800 mg/day.

### Short discussion of the Figures

### Figure 1

A. Correlation between the IC50^{imatinib} and the IUR in 99 patients
B. Box plot demonstrating the difference in IUR between low and high IC50^{imatinib} groups, and also demonstrating the removal of this difference using Prazosin

### Figure 2

A. Correlation between the IC50^{imatinib} and the OCT-1 Activity in 99 patients
B. Box plot demonstrating the difference in OCT-1 Activity between low and high IC50^{imatinib} groups.

### Figure 3

OCT-1 Activity and Molecular response in A. Between low and high OCT-1 Activity groups. B. The effect of dose in patients with high OCT-1 Activity . C. The effect of dose in patients with low OCT-1 Activity

### Figure 4

Kaplan Meier demonstrating the difference in achievement of MMR between low and high OCT-1 Activity groups.

### Figure 5

Demonstrating the effect of dose escalation by dividing patients in to low and high OCT-1 Activity groups and then further subdividing them on the basis of ADD.

### Figure 6

The OCT-1 Activity compared to molecular responses (log reduction in BCR-ABL) at 18 months. Response criteria assessed are suboptimal response (failure to achieve MMR by 18 months), and optimal responses (achievement of 3- to 4-log reduction in BCR-ABL, and >4-log reduction by 18 months. Cohorts are divided:-
A. All patients irrespective of dose received
B. Patients receiving <600mg ADD over the first 12 months
C. Patients who failed to dose escalate to 800mg ADD after 12 months
D. Patients who successfully dose escalated to 800mg ADD after 12 months of therapy

### Figure 7

A. Dot plot showing the correlation between the OCT-1 Activity and OCT-1 mRNA
B. Box plot demonstrating the difference between the levels of OCT-1 mRNA in the low and high OCT-1 Activity groups.

The term "major molecular response (MMR)" as used herein means a 3 log reduction in *BCR*-*ABL* transcripts, quantified from peripheral blood using real-time quantitative reverse-transcriptase polymerase chain reaction, preferably after 12 months of therapy, e.g. 12 months Imatinib mesylate therapy.

The term "complete cytogenic response (CCR)" as used herein means 0 % Philadelphia-chromosome positive metaphases among at least 20 or 25 cells in metaphase in the bone marrow aspirate (Colombat M, Fort MP, Chollet C, et al. Molecular remission in chronic myeloid leukemia patients with sustained complete cytogenetic remission after imatinib mesylate treatment. Haematologica 2006;91:162-8.).

Imatinib is generically and specifically disclosed in the patent applications US 5,521,184, in particular in Example 21, the subject-matter of which is hereby incorporated into the present application by reference. Imatinib can also be prepared in accordance with the processes disclosed in WO03/066613.

For the purpose of the present invention, Imatinib is preferably applied in the form of its mono-mesylate salt. Imatinib mono-mesylate can be prepared in accordance with the processes disclosed in US 6,894,051 the subject-matter of which is hereby incorporated into the present application by reference. Comprised are likewise the corresponding polymorphs, e.g. crystal modifications, which are disclosed therein.

Imatinib mono-mesylate can be administered in dosage forms as described in US 5,521,184, US 6,894,051 or US 2005-0267125.

The collecting of a blood sample from CML patients required under step (a) of the methods described herein can be accomplished by standard procedures being state of the art.

OCT-1 Activity as referred to herein is calculated as the difference in IUR in the absence (total IUR) and presence of prazosin, to provide a measure of the actual activity of the OCT-1 protein in the transport of imatinib. For example: [Total IUR 32ng/200,000 cells)] - [prazosin IUR 23ng/200,000 cells] gives an OCT-1 Activity of 9ng/200,000 cells.

As described in the Examples below, OCT-1 Activity was measured in pre-therapy blood from CML patients by calculating the difference in intracellular uptake and retention (IUR) of [¹⁴C]-Imatinib with and without OCT-1 inhibition. 85% of patients with >median (high) OCT-1 Activity achieved major molecular response (MMR) by 24 months, versus 45% with ≤median (low) OCT-1 Activity. Assessing patients receiving 600mg Imatinib mesylate/day and those averaging <600mg over 12 months of therapy revealed patients with high OCT-1 Activity achieved excellent molecular response regardless of dose, whereas response of patients with low OCT-1 Activity was highly dose dependent. 45% of patients with low OCT-1 Activity who received <600mg failed to achieve a 2-log reduction by 12 months, and 82% failed to achieve a MMR by 18 months, compared to 8% and 17% in the cohort with high OCT-1 Activity and dose <600 mg/day (p=0.017 and p=0.022). OCT-1 Activity is an important determinant of molecular response to Imatinib, and has a predictive value closely linked to dose.

### Examples

The following examples are illustrative, but do not serve to limit the scope of the invention described herein. The examples are meant only to suggest a method of practicing the present invention. The data shown below were collected from samples of CML patients in the TIDEL trial.

### Example 1: Correlation between IC50^{imatinib} and IUR of ¹⁴C imatinib

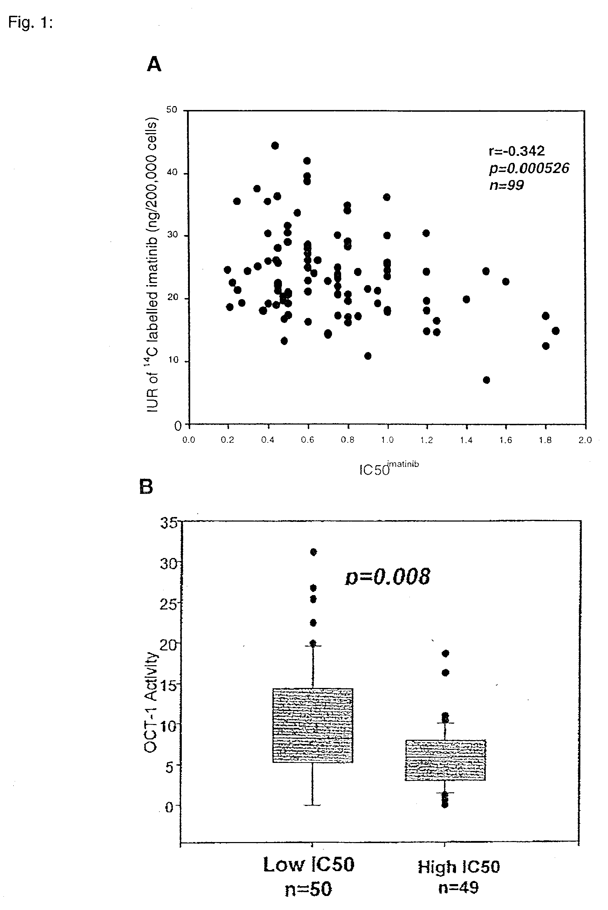

In a smaller series (n=19) a good correlation between the IC50^{imatinib} and the IUR(*p*=*0*.*014*) was demonstrated (White DL, Saunders VA, Dang P, et al. Blood. 2006;108:697-704). In this current expanded series (n=99) again a strong correlation (r = -0.342; p=0.0005) is demonstrated, confirming the relationship between the two parameters (Figure 1A). Furthermore a significant difference between the IUR of the low and high IC50^{imatinib} groups is shown (*p=0.001*), but this difference is removed when the OCT-1 inhibitor prazosin is added (*p=0.129*)(Figure 1 B). This again confirms the importance of the transporter OCT-1 in Imatinib influx.

### Example 2: OCT-1 Activity

The addition of prazosin, a potent inhibitor of OCT-1, to the IUR assay impairs the active transport of Imatinib by OCT-1. Examination of 132 patients enrolled to both trials, reveals a wide variation in OCT-1 Activity (median 8.2: Range 0 to 31.2). In replicate assays of 5 patients the IUR values with prazosin were equal or lower than the values without prazosin. These patients were scored as having negligible (Ong/200,000 cells) OCT-1 Activity.

### Example 3: OCT-1 Activity and IC50^{imatinib}

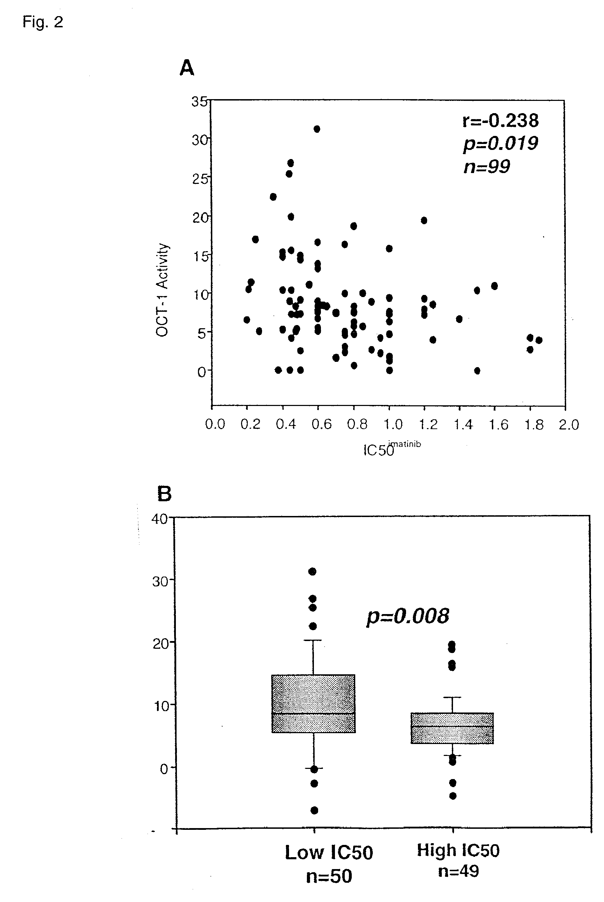

In 99 patients where both IC50^{imatinib} and OCT-1 Activity were measured, there was a significant correlation between the IC50^{imatinib} and the OCT-1 Activity (r = -0.238; *p*=*0.019*) (Fig. 2A). In addition, grouping IC50^{imatinib} into low and high revealed a significantly greater OCT-1 Activity in the low IC50^{imatinib} group when compared to the high (*p*=*0.008*) (Fig 2 B).

### Example 4: OCT-1 Activity, molecular response and the effect of actual dose received

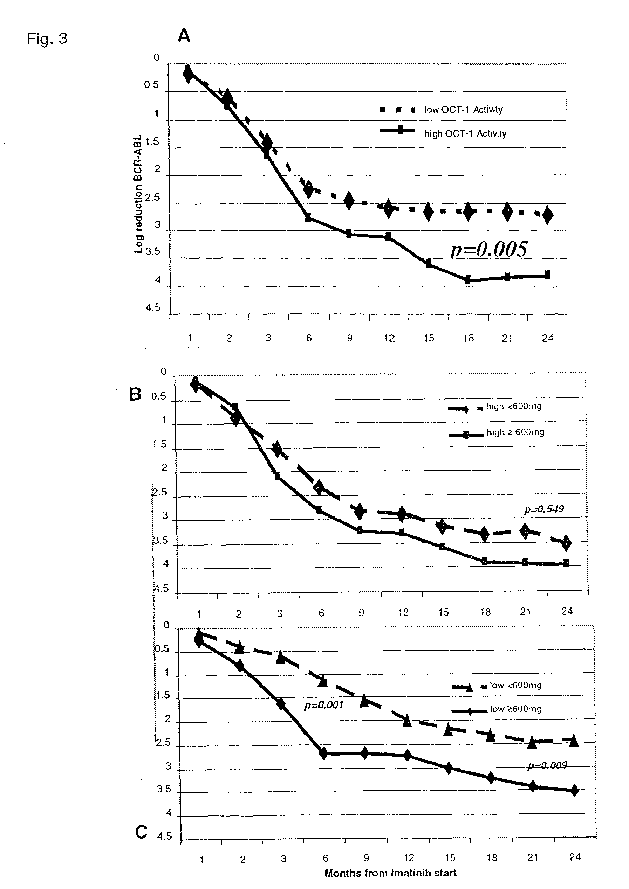

The OCT-1 Activity was compared to molecular response over the first 24 months of Imatinib mesylate therapy in 56 patients enrolled to the TIDEL trial. Patients were grouped into low and high OCT-1 Activity based on the median activity for this cohort of 7.2 ng/200,000 cells. As shown in Table 1 and Figure3A. Patients with high OCT-1 Activity (n=27) achieved significantly higher molecular response over the time course than patients with low OCT-1 Activity (n=29)(*p*=*0.005 at 24 months*).

However, because of tolerability issues, not all patients received 600mg of Imatinib mesylate consistently over the first 12 months of therapy. To assess the effect of varying dose patients were further sub-grouped into those patients who received an average daily dose (ADD) of 600mg (n=33) over the first 12 months of Imatinib therapy, and those who received an ADD of less than 600mg per day (n=23 median ADD 523mg). Four patients with ADD <400mg were included in this cohort.

Assessing only those patients with high OCT-1 Activity there is no significant difference in molecular response between those patients who received <600mg versus those patients who received 600mg or more over the first 12 months *(p=0.449* at 24 months) (Figure 3B, Table 1). In contrast in the cohort of patients with low OCT-1 Activity there is a significant dose effect, with patients receiving 600mg achieving significantly better molecular responses than those who fail to receive 600mg *(p=0.005* at 24 months) (Figure 3B, Table 1).

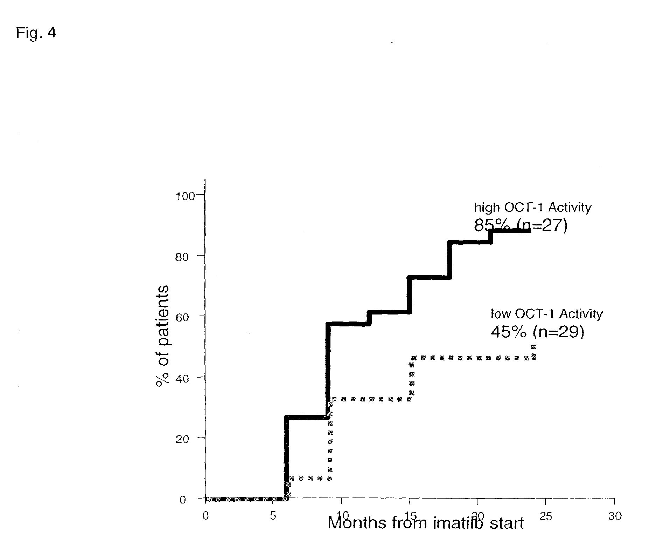

Kaplan Meir Analysis revealed 85% of patients with high OCT-1 Activity achieved a MMR (median time to achieve 9 months) by 24 months versus only 45% of patients with low OCT-1 Activity (median time 24 months) p=0.009 (Figure 4). Analysing only those patients who received <600mg ADD over the first 12 months, reveals a significant effect of dose between low and high OCT-1 Activity patients: 83% of patients with high OCT-1 Activity (n=12) achieve MMR, compared to 18% of patients with a low OCT-1 Activity (n=11) p=0.022. There was no significant difference however between the groups in that cohort of patients receiving 600mg (p=0.110)

**Table 1 OCT-1 Activity and MR**

| | **Average Molecular Response at 6 monthly Intervals** | | |
|---|---|---|---|
| **OCT-1 Activity** | **12** | **18** | **24** |
| low (n=29) | 2.6 | 2.6 | 2.8 |
| High (n=27) | 3.1 | 3.9 | 3.9 |
| *p-value* | *0. 032* | *0. 006* | *0. 005* |

| **Low OCT-1 Activity** | | | |
|---|---|---|---|
| <600mg (n-11) | 2.1 | 2.3 | 2.4 |
| ≥600mg(n=18) | 2.8 | 3.2 | 3.4 |
| *p- value* | *0.121* | *0.023* | *0.005* |

| **High OCT-1 Activity** | | | |
|---|---|---|---|
| <600mg (n=12) | 2.9 | 3.3 | 3.5 |
| ≥600mg (n=15) | 2.9 | 3.9 | 3.9 |
| *p- value* | *0.789* | *0.625* | *0.449* |

### Example 5: Effect of dose increase

In the TIDEL trial, dose increases from the initial 600mg to 800 mg per day were mandated if a 4 log reduction in BCR-ABL from the standardized baseline was not achieved by 12 months. In the patient cohort of the present Example 46 patients were scheduled to receive dose increases. In this analysis a dose increase was considered to have occurred if the patient received 800mg ADD for at least one month.

For reasons of toxicity only 29 (63%) patients in this cohort successfully dose increased to 800mg per day. The primary reason for inability was previous toxicity/tolerance issues which occurred in 13 of those 17 patients who did not dose escalate. Of the remaining 4 patients 2 dose escalated for one month but did not reach 600mg ADD. It is not known why the remaining 2 patients failed to escalate.

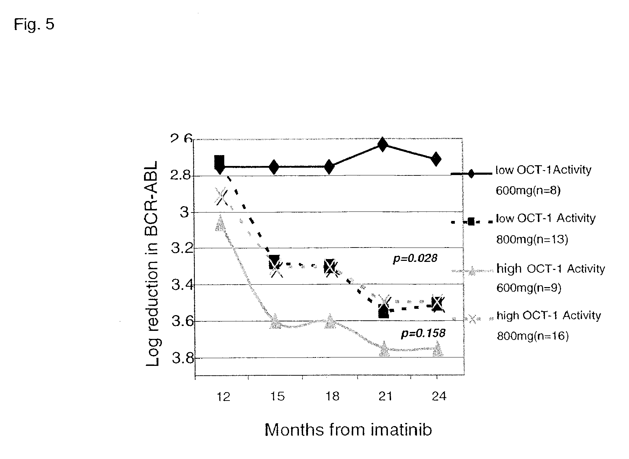

Dose increase occurred by 14 months in all patients who were able to dose increase. To assess the impact of dose escalation patients were grouped into low and high OCT-1 Activity groups as previously, then were further subdivided into those who received increased dose and those who remained on 600mg or less as shown in Figure 5 there was no significant difference between the patients who dose increased and the patients who failed to dose increase in the high OCT-1 Activity cohort (p>0.05 at all time points). In contrast, patients with a low OCT-1 Activity perform equally well as those patients with high Activity, when they are dose increased. However those who fail to dose increase had significantly inferior molecular responses at 24 months (Figure 5).

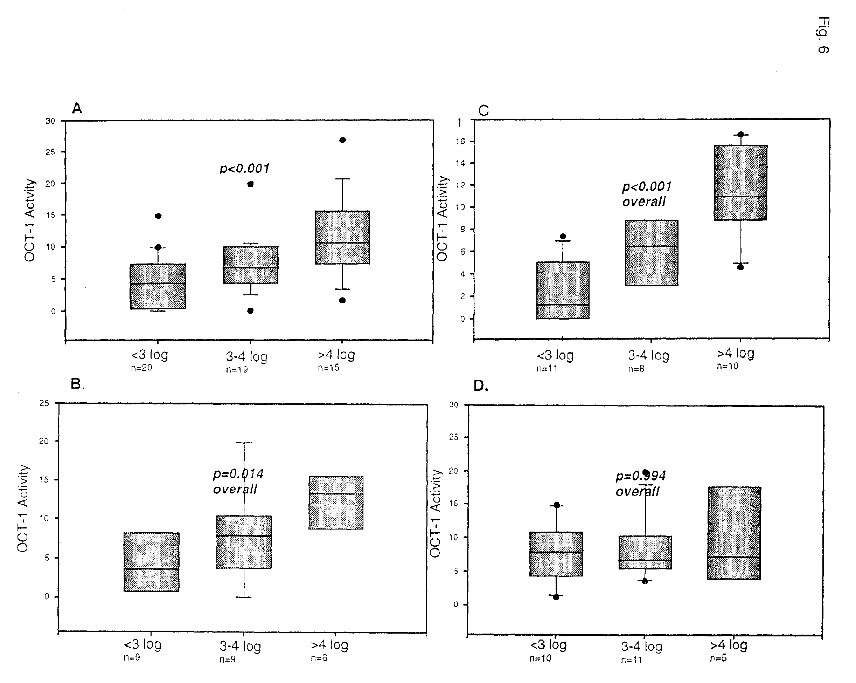

The median OCT-1 Activity in patients who failed to achieve MMR by 18 months is significantly lower than OCT-1 Activity in patients who did achieve MMR (Figure 6). These differences in OCT-1 activity between molecular response groups is most pronounced in patients who were not able to dose escalate at 12 months (Figure 6c). In patients who dose increased at 12 months there is no significant difference between the 3 groups (Figure 6d).

### Example 6: OCT-1 Activity, Suboptimal Response and Imatinib Failure

Suboptimal response has been defined as failure to achieve a major cytogenetic response by 6 months, a complete cytogenetic response by 12 months or a MMR by 18 months (Baccarani M, Saglio G, Goldman J, et al. Blood. 2006;108:1809-1820). As good correlation between molecular and cytogenetic response has been demonstrated previously (Branford S, Hughes TP, Rudzki Z. Br J Haematol. 1999;107:587-599) the suboptimal response as failure to achieve 1-log reduction in BCR-ABL by 6 months, a 2-log reduction by 12 months and a 3-log reduction (MMR) by 18 months was assessed. Kaplan Meier analysis as reported in Table 2 reveals there is no significant difference in the frequency of suboptimal response at any time-point when patients receive 600mg ADD, however there is a significant difference at all time-points when patients receive reduced dose, demonstrating patients with low OCT-1 Activity who receive reduced dosing are at substantial risk of suboptimal response to Imatinib mesylate.
Assessing the failure to achieve a 2-log reduction by 18 months (Imatinib failure, Baccarani M, Saglio G, Goldman J, et al. see above) reveals also that a significantly lower proportion of patients with low OCT-1 Activity who receive reduced dose achieve a 2-log reduction by 18 months (high OCT-1 Activity 8% low OCT-1 Activity 36% *p-0.04).*

**Table 2 Sub optimal response**

| | % of patients failing to achieve | | |
|---|---|---|---|
| | 1 log reduction in BCR-ABL by 6 months | 3 log reduction in BCR-ABL by 12 months | 3 log reduction in BCR-ABL by 18 months |
| | Patients receiving = 600mg ADD | | |
| high OCT-1 Activity (n=15) | 7% | 13% | 20% |
| low OCT-1 Activity (n=18) | 0% | 0% | 44% |
| *P value* | *0.669* | *0.560* | *0.133* |

| | Patients receiving < 600mg ADD | | |
|---|---|---|---|
| high OCT-1 Activity (n=15) | 0% | 8% | 17% |
| low OCT-1 Activity (n=18) | 27% | 45% | 82% |
| *P value* | ***0.005*** | ***0.021*** | ***0.022*** |

### Example 7: OCT-1 mRNA and OCT-1 Activity

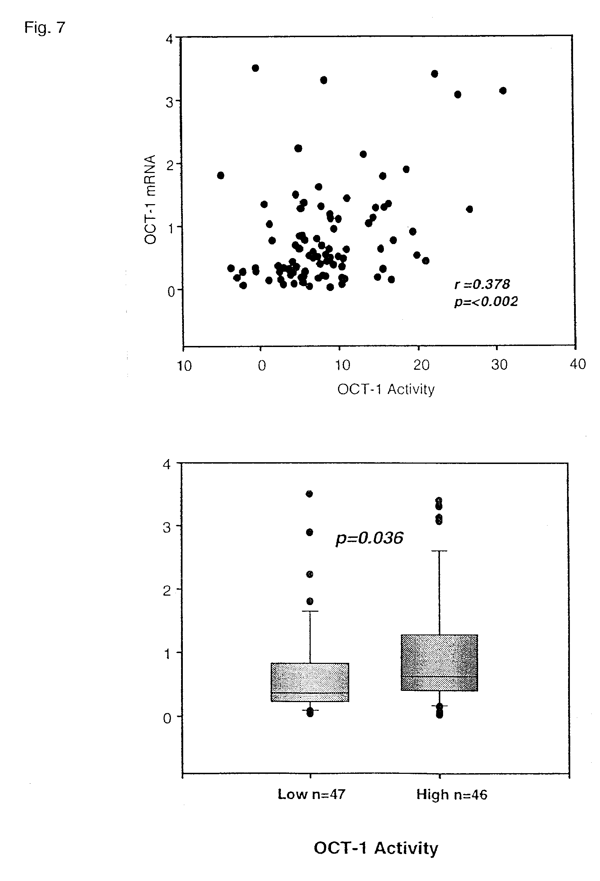

The level of OCT-1 mRNA was measured relative to BCR mRNA in 93 patients. The relative mean % mRNA was 0.835 within a range 0.02 to 3.5%. Correlating mRNA with OCT-1 Activity revealed a p-value of 0.002 (r= 0.378) (Figure 7). Dividing patients into low and high OCT-1 Activity about the median for this cohort of 7.9, reveals a significant difference between the two groups. The median % OCT-1 mRNA for patients with low OCT-1 Activity (n=47) was 0.367 compared to 0.635 (n=46) for patients with high OCT-1 Activity (p=0.036) Figure 7.

Interestingly, in the 77 patients where IC50 and OCT-1 mRNA analyses were available, there was no correlation (*r=0.162; p=0.171*) between the two parameters. Further grouping the patients into low and high IC50^{imatinib} and assessing the level of OCT-1 mRNA expression revealed no significant difference between the two groups (low IC50^{imatinib} n=42, median 0.586; high IC50^{imatinib} n=35 median 0.546; *p=0.570)*

Limiting analysis to those patients where 24 month molecular follow-up is available (TIDEL patients) a significant difference in the molecular response between the two groups (low and high OCT-1 mRNA) could not be demonstrated (Table 3). Also in contrast to OCT-1 Activity there was no significant difference between the two groups with respect to dose (600mg or lower), and dose escalation (to 800mg) (Table 3) Unlike OCT-1 Activity, mRNA analysis did not reveal a group of patients with suboptimal response or at risk for Imatinib failure (data not shown).

**Table 3. OCT-1 mRNA and MR**

| | **Average Molecular Response at 6 monthly Intervals** | | |
|---|---|---|---|
| **mRNA** | **12** | **18** | **24** |
| low (n=22) | 2.9 | 3.0 | 3.3 |
| high (n=21) | 2.9 | 3.9 | 3.9 |
| *p-value* | 0.693 | 0.09 | *0.125* |

| **Low OCT-1 mRNA** | | | |
|---|---|---|---|
| <600mg (n=10) | 2.8 | 2.9 | 2.9 |
| ≥600mg (n=12) | 2.8 | 3.1 | 3.3 |
| *p- value* | *0.971* | *0.485* | *0.402* |

| **High OCT-1 mRNA** | | | |
|---|---|---|---|
| <600mg (n=8) | 2.5 | 3.6 | 3.7 |
| ≥600mg (n=13) | 2.9 | 3.9 | 3.9 |
| *p- value* | *0.337* | *0.856* | *0.8* |

## Claims

1. Method of treating chronic myeloid leukemia (CML) in a warm-blooded animal comprising the steps of
(a) determining the OCT-1 Activity in pre-therapy blood of a warm-blooded animal suffering from CML, and
(b) administering a daily dose between about 500 and 1200 mg of Imatinib mesylate to the warm-blooded animal suffering from CML showing an OCT-1 Activity below about 6.0 to 10.0 ng/200,000 cells, especially about 8.0 to 8.5 ng/200,000 cells.

2. Method according to claim 1 wherein the warm blooded animal is a human.

3. Method according to claim 2 wherein in step (a) a daily dose of between about 600 and 1000 mg of Imatinib mesylate is administered orally.

4. Method according to claim 2 wherein in step (a) a daily dose of between about 600 mg of Imatinib mesylate is administered orally.

5. Method according to any one of claims 1 to 4 wherein the warm-blooded animal suffering from CML is showing an OCT-1 Activity below about 8.0 to 8.5 ng/200,000 cells.
